# EUROPEAN PATENT SPECIFICATION

(11) **EP 3 813 566 B1**
(45) Date of publication and mention of the grant of the patent: **02.09.2026**
(21) Application number: 19734773.5
(22) Date of filing: 28.06.2019
(51) Int. Cl.: A24B 3/14, A24C 5/01, A24D 1/20, A61K 36/81, A24B 15/12, A24B 15/24

(54) **A METHOD FOR MANUFACTURING RECONSTITUTED PLANT MATERIAL**
VERFAHREN ZUR HERSTELLUNG VON REKONSTITUIERTEM PFLANZENMATERIAL
PROCÉDÉ DE FABRICATION DE MATÉRIAU VÉGÉTAL RECONSTITUÉ

(30) Priority: 29.06.2018 GB 201810727; 29.06.2018 GB 201810726
(43) Date of publication of application: 05.05.2021
(73) Proprietor: Imperial Tobacco Limited, Bristol, BS3 2LL (GB)
(72) Inventor: FERRIE, Kate, Liverpool Merseyside L24 9HP (GB); SHENTON, Edward Ross, Liverpool Merseyside L24 9HP (GB)
(74) Representative: Haseltine Lake Kempner LLP
(86) International application number: PCT/EP2019/067307
(87) International publication number: WO 2020/002585

(56) References cited:
- WO-A1-2017/097840
- WO-A1-2017/181684
- WO-A1-2017/181684
- US-A- 5 325 877
- US-A1- 2005 056 294
- US-A1- 2008 216 854
- US-A1- 2011 247 640
- US-A1- 2015 020 824
- DATABASE WPI Week 201671, Derwent World Patents Index; AN 2016-45269N
- DATABASE WPI Week 201671, Derwent World Patents Index; AN 2016-45269N

## Description

### Field of the Invention

The present invention relates to a method for reconstituting a plant material by a paper-making process, and a reconstituted tobacco product.

### Background

During the manufacturing process for tobacco products, such as cigarettes, tobacco waste is produced. The tobacco waste is typically in the form of tobacco dust, leaf or stem. The tobacco waste can be collected and processed to provide what is known as reconstituted tobacco, or "recon".

Two distinct methods are most commonly employed to manufacture reconstituted tobacco, these are i) a paper-making process, and ii) slurry casting. The products that result from these distinct processes have notably different properties and characteristics. In particular, recon formed by the paper-making process typically has a reduced aroma/flavour and nicotine content in comparison to that formed by slurry casting.

In slurry casting the tobacco waste is milled to a fine powder and mixed with an aqueous solvent, typically water. The resultant slurry may undergo further milling to reduce the particle size of the tobacco material further. The slurry is then cast on a surface and dried to form a sheet. The dried sheet may be shredded to be used within various tobacco products, for example as a cigarette filler.

In the paper-making process the tobacco waste is mechanically beaten in the presence of an aqueous solvent to digest and process the tobacco into workable fibres to be made into a paper or web. Subsequently, water soluble compounds present in the tobacco are extracted into the aqueous solvent. The aqueous extract and insoluble fibrous portion are separated. The separated fibrous portion, which may also be described as a "pulp", undergoes further processing to form a base sheet via a typical paper-making procedure. The aqueous extract is concentrated and then reapplied to the base sheet. The base sheet is then dried to form a paper.

The paper formed by the above process can be shredded and used as a filler within typical smoking articles such as cigarettes. Alternatively, it may be used as a wrapper to contain a tobacco rod. Conventional reconstituted tobacco paper of this kind is generally not suitable for "heat-not-burn" ("HNB") devices. For recon paper to be suitable for use within a HNB device a humectant must also be incorporated. Typical humectants include propylene glycol ("PG") or vegetable glycerine ("VG").

An HNB device heats the tobacco without burning it, i.e. it does not undergo combustion. The purpose of HNB devices is to avoid production, and subsequent inhalation, of harmful combustion products such as tar. By heating a humectant-impregnated recon paper the humectant is vaporised to form an aerosol, also referred to as a "vapour". The aerosol may contain aroma/flavour compounds and/or nicotine derived from the recon paper. It is the aerosol which enables the user to simulate the act of smoking. WO 2017/051034 describes a reconstituted tobacco made by a paper making process which is suitable for an HNB device. In which, a humectant is added to the concentrated tobacco extract before application to the base-sheet to provide a humectant-impregnated recon paper.

Associated with the recon paper-making process is the loss of volatile compounds, such as nicotine and flavour compounds, from the tobacco during processing. This is linked with the large volumes of water used to digest and process the tobacco, and the high temperatures used to evaporate the water.

It is desirable to provide a safe and efficient way of increasing the nicotine and/or flavour content of recon tobacco made by a paper-making process. In particular it is desirable to provide a product that delivers an enhanced sensory experience to the consumer, which is consistent over multiple consumables.

Various efforts have been made to improve the nicotine content of recon tobacco made by a paper-making process. In US 3422819, the nicotine content of a cigarette wrapper was increased by applying a solution of nicotine salt to a reconstituted tobacco sheet after manufacture of the paper. Alternatively, it is recognised in US 3422819 that "the solution of nicotine salt may be added to the stock from which paper is formed". The term "stock" refers to the mixture of pulp, fillers, other papermaking materials and water (see C. Biermann, Handbook of Pulping and Papermaking, 2nd Edition). The stock, therefore, is the fibrous mixture post-extraction that enters the paper-making machine to be formed into a base sheet.

US 3,861,400 describes a method of increasing the nicotine content of recon tobacco formed by a slurry casting method. This is achieved by the addition of specific polyuronic acid nicotine salt derivatives to a tobacco-water slurry. The products produced by slurry casting, and the method itself, are distinct to that of the recon paper-making process.

GB 1466912 describes an alternative method of increasing the nicotine content within recon tobacco paper. In which, roots of plants of the genus *Nicotiana* are grown in a specific culture medium. The grown roots are combined with tobacco solids to form a reconstituted tobacco base sheet. The spent culture medium, which includes nicotine exuded by the roots, is added to the tobacco soluble obtained in the production of the reconstituted tobacco. The tobacco soluble is concentrated and added to the base sheet.

CN 103468403 and CN 103462204 describe a method for extracting tobacco aroma compounds by conducting two separate extraction processes directly upon the tobacco raw material. This includes separate water extraction and supercritical carbon dioxide (scCO₂) extraction on the tobacco raw material. The two extracts are then combined and applied to the reconstituted tobacco.

Alternatively, CN 103783660 describes scCO₂ extraction on tobacco leaf fragments to extract flavour compounds. The flavour compounds are then separated, purified and reapplied to a reconstituted tobacco product by spraying or coating.

WO2017/181684 concerns a method for manufacturing a heat-not-burn tobacco cigarette. US2011/247640 concerns a smokeless tobacco product configured for insertion into the mouth of a user and incorporates tobacco-derived materials and non-tobacco plant materials. WO 2017/097840 concerns a tobacco composition comprising a tobacco component in an amount of from 60 to 90% by weight of the tobacco composition, a filler in an amount of 0 to 20% by weight of the tobacco composition, and an aerosol generating agent in an amount of from 10 to 20% by weight of the tobacco composition.

In the context of reconstituted tobacco suitable for use within a HNB device a number of documents describe that flavour additives may be optionally added to the humectant-impregnated recon paper, see for example, WO2017/051034, US 5325877, US 5715844 and US 2006/0021626. However, it is not specified within these documents how the flavour additives are added, nor at what stage in the paper-making process the flavour additives are added. Indeed, it is not recognised that flavour compounds are lost during the recon paper-making process. Therefore, it is apparent that the flavour additives are not intended to replace lost flavour during processing, but instead to impart a different taste experience to that of tobacco.

The present invention has been devised in light of the above considerations.

### Summary of the Invention

The invention is defined by the appended set of claims.

### First Mode: Addition of Flavour

This mode is provided for reference only and does not form part of the claimed invention. According to a first aspect of the first mode, there is provided a method for manufacturing a reconstituted plant material, the method comprising:
extracting a fibrous plant material with a solvent to provide an extract and a fibrous portion;
separating the extract from the fibrous portion;
concentrating the extract to provide a concentrated extract;
forming a base sheet from the fibrous portion;
combining a flavouring contained within a humectant with the concentrated extract;
applying the concentrated extract to the base sheet.

Nicotine is a highly volatile compound and a controlled substance, consequently a number of handling difficulties exist during processing, as it is the subject of strict exposure limits. Similarly, many flavour compounds are volatile and are also the subject of exposure limits.

Advantageously, the pre-mixing of a flavouring and humectant prior to combining with the concentrated extract makes for a safer, efficient and reproducible delivery of flavouring to the concentrated extract. Additionally, by including a flavouring addition step in-situ to the paper-making process, flavour losses during processing are negated (e.g. losses through evaporation and high dilution). Furthermore, combining a mixture of flavouring and humectant to the concentrated extract ensures improved impregnation and uniform distribution of the flavouring and humectant throughout the final product. This provides a more consistent and improved sensory experience to the user.

Also, by providing the flavouring and humectant to the concentrated extract (which also contains other soluble compounds such as nicotine derived from the plant material) a homogeneous mixture of nicotine, humectant and flavour compounds is obtained. Consequently, a greater degree of intermingling of nicotine, humectant and flavour compounds is achieved in the recon paper to provide an improved sensory experience to the user.

During extraction the mixture of fibrous plant material and solvent are mechanically beaten to form a pulp. A pulp consists of lignocellulosic materials, e.g. plant fibres, which have been broken down physically and/or chemically such that discrete fibres are liberated and can be dispersed in solvent and reformed to form a web. Optionally, additional chemicals may be added to the mixture to aid digestion of the tobacco material and pulp formation. Suitable chemicals are known to those skilled in the art.

Suitable solvents are known to those skilled in the art. These include non-aqueous solvents and aqueous solvents. Examples of non-aqueous solvents includes but is not limited to alcohols, such as ethanol and propanol; acetone; acetonitrile; benzene; dichloromethane; ethyl acetate; hexane; and toluene.

Preferably, the solvent is an aqueous solvent. More preferably, the solvent is water. Optionally, the aqueous solvent may contain a co-solvent. Preferably, the co-solvent is water miscible. Examples of co-solvents are alcohols, acetone and acetonitrile.

Advantageously, water is a non-toxic and non-flammable solvent and is thus is easier to handle during processing. Furthermore, the risk of toxic solvent residues remaining in the final product is removed.

The temperature of the tobacco-water mixture during extraction is generally within the range 10 - 100 °C. Preferably, the temperature is within the range 30 - 90 °C. Particularly, preferred is the range 30 - 70 °C.

The extraction time generally ranges from 30 minutes to 6 hours. Preferably, the extraction time is less than 4 hours, such as less than 3 hours, or such as less than 2 hours. Particularly, preferred is an extraction time less than 1 hour.

Preferably, the fibrous plant material is tobacco.

Advantageously, as the first mode is primarily intended to provide a smoking consumable, the use of tobacco provides a reconstituted plant material product having the inherent properties of tobacco, for example the texture, aroma and nicotine content.

Preferably, the tobacco is in the form of tobacco leaf, tobacco stem, tobacco powder and/or tobacco dust.

Advantageously, these forms of tobacco are typically derived as waste from tobacco processing, thus by utilising this "waste" to form the product of the first mode the amount of unused tobacco material is minimised.

Preferably, the flavouring is selected from Mild Menthol, Mixed Berry, Cool Menthol, Peach, Cream Cake, Melon Medley, Apple, Shisha Grape, Virginia Tobacco, and American Red Blend.

Advantageously, these flavourings provide the user with a satisfactory flavour "hit" and additionally provide an improved sensory experience to the user.

Preferably, the humectant is a polyol.

Advantageously, good dispersion of flavouring in the humectant is achieved when the humectant is a polyol.

Preferably, the polyol is propylene glycol or vegetable glycerine or a combination thereof.

Advantageously, both propylene glycol and vegetable glycerine exhibit minimal toxicity. Furthermore, they impart a sweet taste that is satisfactory to the user.

Preferably, the flavouring content contained within the humectant is within the range 1 to 95 wt % based on the total weight of flavouring and humectant, such as 1 to 50 wt %, such as 1 to 25 wt %, such as 1 to 15 wt %.

Advantageously, this provides sufficient dilution of flavouring with humectant to provide safer handling of flavouring during processing. Also, such a combination allows for good dispersion of flavouring within the humectant.

The method according to the first aspect of the first mode may further comprise a step of making an HNB consumable.

According to a second aspect of the first mode, there is provided a reconstituted tobacco product for an HNB device produced by a process according to the first aspect of the first mode comprising:
a humectant;
and a flavouring;
wherein the humectant content is 1 to 50 % by weight of the reconstituted tobacco product,
and wherein the flavouring content is 2 to 25 % by weight of the reconstituted tobacco product.

Advantageously, by increasing the minimum amount of flavouring present in the reconstituted tobacco product the vapour produced, when in use, will have a higher flavour content; thus a stronger flavour "hit" may be experienced by the user. In addition, the size of the product itself may be decreased, in comparison to known recon paper products, without any reduction in the total amount of flavour delivered to the user per consumable.

Preferably, the humectant is a polyol.

Preferably, the polyol is propylene glycol or vegetable glycerin or combinations thereof.

The advantages of using a polyol humectant, and in particular, propylene glycol and vegetable glycerine is as described for the first aspect of the first mode.

Preferably, the flavouring is selected from Mild Menthol, Mixed Berry, Cool Menthol, Peach, Cream Cake, Melon Medley, Apple, Shisha Grape, Virginia Tobacco, and American Red Blend.

The advantages of using flavourings from this selection is as described for the first aspect of the first mode.

The flavouring content of the reconstituted tobacco product may have a lower limit of at least 2 wt % of the weight of the reconstituted tobacco product, such as at least 3 wt %, such as at least 4 wt %, such as at least 5 wt %, such as at least 10 wt %, such as at least 15 wt %, such as at least 20 wt %.

The flavouring content of the reconstituted tobacco product may have an upper limit of at most 25 wt %, such as at most 20 wt %, such as at most 15 wt %, or such as at most 10 wt %.

Preferably, the flavouring content is 5 to 25 wt % of the reconstituted tobacco product, such as 10 to 25 wt %, such as 15 to 25 wt %

Advantageously, a reconstituted tobacco product with flavour content higher than achieved using typical processes is provided. This enables the amount of flavour to be tailored to suit the user's requirements.

The humectant content of the reconstituted tobacco product may have a lower limit of at least 1 % by weight of the reconstituted tobacco, such as at least 2 wt %, such as at least 5 wt %, such as at least 10 wt %, such as at least 20 wt %, such as at least 30 wt %, or such as least 40 wt %.

The humectant content of the reconstituted tobacco product may have an upper limit of at most 50 % by weight of the reconstituted tobacco, such as at most 40 wt %, such as at most 30 wt %, or such as at most 20 wt %.

Preferably, the humectant content is 1 to 40 wt % of the reconstituted tobacco product, such as 1 to 20 wt %

Advantageously, an amount of humectant within this range provides the user with a satisfactory "hit" of vapour and additionally provides the user with an improved sensory experience.

According to a third aspect of the first mode, there is provided use of the reconstituted tobacco product according to the second aspect of the first mode in a HNB device.

According to a fourth aspect of the first mode, there is provided an HNB consumable comprising the reconstituted tobacco product according to the second aspect of the first mode.

According to a fifth aspect of the first mode, there is provided an HNB system comprising an HNB consumable according to the fourth aspect of the first mode, and an HNB device.

According to a sixth aspect of the first mode, there is provided a reconstituted tobacco product comprising:
a humectant;
and a flavouring;
wherein the humectant content is 1 to 50 % by weight of the reconstituted tobacco product,
and wherein the flavouring content is 2 to 25 % by weight of the reconstituted tobacco product.

### Second Mode of Invention: Addition of Nicotine

This mode is in accordance with the claimed invention. According to a first aspect of the second mode of invention, there is provided a method for manufacturing a reconstituted plant material, the method comprising:
extracting a fibrous plant material with a solvent to provide an extract and a fibrous portion;
separating the extract from the fibrous portion;
concentrating the extract to provide a concentrated extract;
forming a base sheet from the fibrous portion;
providing a pre-mixture by combining nicotine with a humectant
combining the pre-mixture with the concentrated extract to provide a modified extract;
applying the modified extract to the base sheet.

Nicotine is a highly volatile compound and a controlled substance, consequently a number of handling difficulties exist during processing, as it is the subject of strict exposure limits. Similarly, many flavour compounds are volatile and are also the subject of exposure limits.

Advantageously, the pre-mixing of nicotine and humectant prior to combining with the concentrated extract makes for a safer, efficient and reproducible delivery of nicotine to the concentrated extract. Additionally, by including a nicotine addition step in-situ to the paper-making process, nicotine losses during processing are negated (e.g. losses through evaporation and high dilution). Furthermore, combining a mixture of nicotine and humectant to the concentrated extract ensures improved impregnation and uniform distribution of the nicotine and humectant throughout the final product. This provides a more consistent and improved sensory experience to the user.

Also, by providing the nicotine and humectant to the concentrated extract (which also contains other soluble compounds such as flavour compounds derived from the plant material) a homogeneous mixture of nicotine, humectant and flavour compounds is obtained. Consequently, a greater degree of intermingling of nicotine, humectant and flavour compounds is achieved in the recon paper to provide an improved sensory experience to the user.

During extraction the mixture of fibrous plant material and solvent are mechanically beaten to form a pulp. A pulp consists of lignocellulosic materials, e.g. plant fibres, which have been broken down physically and/or chemically such that discrete fibres are liberated and can be dispersed in solvent and reformed to form a web. Optionally, additional chemicals may be added to the mixture to aid digestion of the tobacco material and pulp formation. Suitable chemicals are known to those skilled in the art.

Suitable solvents are known to those skilled in the art. These include non-aqueous solvents and aqueous solvents. Examples of non-aqueous solvents includes but is not limited to alcohols, such as ethanol and propanol; acetone; acetonitrile; benzene; dichloromethane; ethyl acetate; hexane; and toluene.

Preferably, the solvent is an aqueous solvent. More preferably, the solvent is water. Optionally, the aqueous solvent may contain a co-solvent. Preferably, the co-solvent is water miscible. Examples of co-solvents are alcohols, acetone and acetonitrile.

Advantageously, water is a non-toxic and non-flammable solvent and is thus is easier to handle during processing. Furthermore, the risk of toxic solvent residues remaining in the final product is removed.

The temperature of the tobacco-water mixture during extraction is generally within the range 10 - 100 °C. Preferably, the temperature is within the range 30 - 90 °C. Particularly, preferred is the range 30 - 70 °C.

The extraction time generally ranges from 30 minutes to 6 hours. Preferably, the extraction time is less than 4 hours, such as less than 3 hours, or such as less than 2 hours. Particularly, preferred is an extraction time less than 1 hour.

Preferably, the fibrous plant material is tobacco.

Advantageously, as the invention is primarily intended to provide a smoking consumable, the use of tobacco provides a reconstituted plant material product having the inherent properties of tobacco, for example the texture, aroma and nicotine content.

Preferably, the tobacco is in the form of tobacco leaf, tobacco stem, tobacco powder and/or tobacco dust.

Advantageously, these forms of tobacco are typically derived as waste from tobacco processing, thus by utilising this "waste" to form the product of the invention the amount of unused tobacco material is minimised.

Preferably, the humectant is a polyol.

Advantageously, good dispersion of nicotine in the humectant is achieved when the humectant is a polyol.

Preferably, the polyol is propylene glycol or vegetable glycerine or a combination thereof.

Advantageously, both propylene glycol and vegetable glycerine exhibit minimal toxicity. Furthermore, they impart a sweet taste that is satisfactory to the user.

Preferably, the nicotine content contained within the humectant is within the range 1 to 95 wt % based on the total weight of nicotine and humectant, such as 1 to 50 wt %, such as 1 to 25 wt %, such as 1 to 15 wt %

Advantageously, this provides sufficient dilution of nicotine with humectant to provide safer handling of nicotine during processing. Also, such a combination allows for good dispersion of nicotine within the humectant.

The method according the first aspect of the second mode of invention may further comprise a step of making an HNB consumable.

According to a second aspect of the second mode of the invention, there is provided a reconstituted tobacco product for an HNB device produced by a process according the first aspect of the second mode of invention comprising:
a humectant;
and nicotine;
wherein the humectant content is 1 to 50 % by weight of the reconstituted tobacco product,
and wherein the nicotine content is 2 to 25 % by weight of the reconstituted tobacco product.

Advantageously, by increasing the minimum amount of nicotine present in the reconstituted tobacco product the vapour produced, when in use, will have a higher nicotine content; thus a stronger nicotine "hit" may be experienced by the user. In addition, the size of the product itself may be decreased, in comparison to known recon paper products, without any reduction in the total amount of nicotine delivered to the user per consumable.

Preferably, the humectant is a polyol.

Preferably, the polyol is propylene glycol or vegetable glycerin or combinations thereof.

The advantages of using a polyol humectant, and in particular, propylene glycol and vegetable glycerine is as described for the first aspect of the second mode of invention

The nicotine content of the reconstituted tobacco product may have a lower limit of at least 2 wt % of the weight of the reconstituted tobacco product, such as at least 3 wt %, such as at least 4 wt %, such as at least 5 wt %, such as at least 10 wt %, such as at least 15 wt %, such as at least 20 wt %.

The nicotine content of the reconstituted tobacco product may have an upper limit of at most 25 wt %, such as at most 20 wt %, such as at most 15 wt %, or such as at most 10 wt %.

Preferably, the nicotine content is 5 to 25 wt % of the reconstituted tobacco product such as 10 to 25 wt %, such as 15 to 25 wt %

Advantageously, a reconstituted tobacco product with nicotine content higher than achieved using typical processes is provided. This enables the amount of nicotine to be tailored to suit the user's requirements.

The humectant content of the reconstituted tobacco product may have a lower limit of at least 1 % by weight of the reconstituted tobacco, such as at least 2 wt %, such as at least 5 wt %, such as at least 10 wt %, such as at least 20 wt %, such as at least 30 wt %, or such as least 40 wt %.

The humectant content of the reconstituted tobacco product may have an upper limit of at most 50 % by weight of the reconstituted tobacco, such as at most 40 wt %, such as at most 30 wt %, or such as at most 20 wt %.

Preferably, the humectant content is 1 to 40 wt % of the reconstituted tobacco product, such as 1 to 20 wt %

Advantageously, an amount of humectant within this range provides the user with a satisfactory "hit" of vapour and additionally provides the user with an improved sensory experience.

According to a third aspect of the second mode of invention, there is provided use of the reconstituted tobacco product according to the second aspect of the second mode of invention in a HNB device.

According to a fourth aspect of the second mode of invention, there is provided an HNB consumable comprising the reconstituted tobacco product according to the second aspect of the second mode of invention.

According to a fifth aspect of the second mode of invention, there is provided an HNB system comprising an HNB consumable according to the fourth aspect of the second mode of the invention, and an HNB device.

According to a sixth aspect of the second mode of invention, there is provided reconstituted tobacco product comprising:
a humectant;
and nicotine;
wherein the humectant content is 1 to 50 % by weight of the reconstituted tobacco product,
and wherein the nicotine content is 2 to 25 % by weight of the reconstituted tobacco product.

### Brief Description of the Drawings

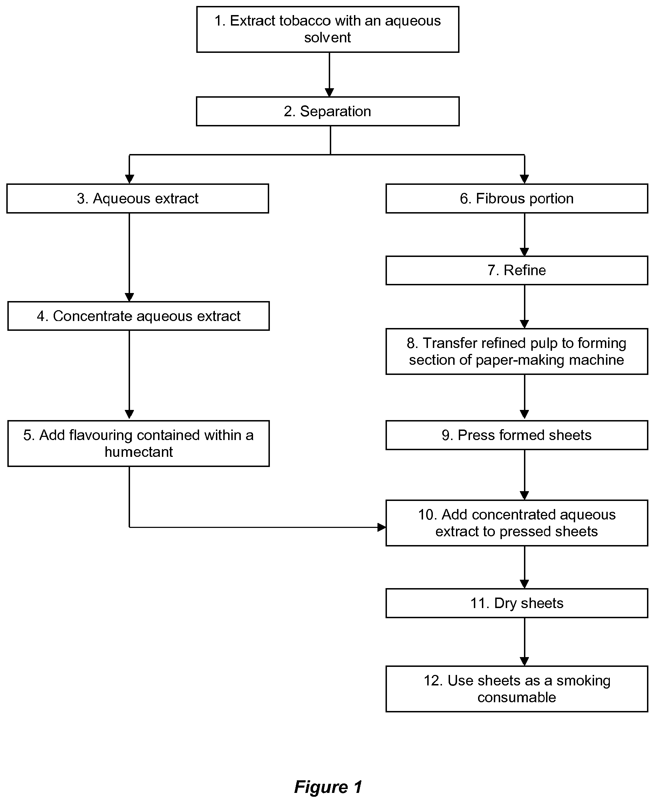

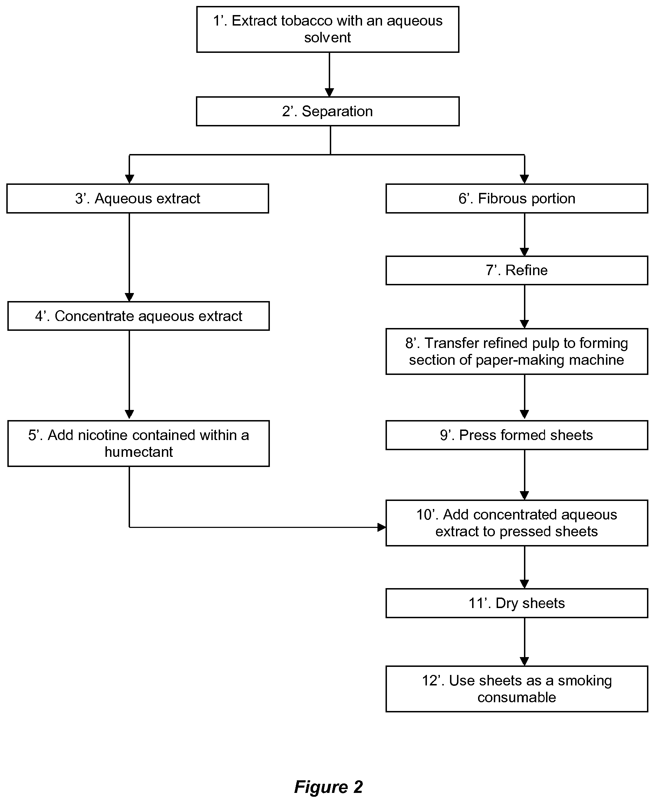

A complete understanding of preferred embodiments of the present invention may be obtained by reference to the accompanying drawing in Figure 2, when considered in conjunction with the subsequent, detailed description, provided by way of example only. Figure 1 relates to the first mode, which does not form part of the claimed invention, and is provided for reference only.
Figure 1 is an illustrative view of a manufacturing process for producing reconstituted tobacco for use in accordance with the first mode.
Figure 2 is an illustrative view of a manufacturing process for producing reconstituted tobacco for use in accordance with the second mode of the present invention.

### Detailed Description of the Invention

Unless defined otherwise, all technical and scientific terms used herein have the same meaning as commonly understood by one of ordinary skill in the art to which this invention belongs or as determined by the context in which such terms are used. Although any methods and materials similar or equivalent to those described herein can also be used in the practise or testing of the present invention, a limited number of exemplary methods and materials are described herein.

The publications discussed herein are provided solely for their disclosure prior to the filing date of the present patent application.

### First Mode: Addition of Flavour

This mode is provided for reference only and does not form part of the claimed invention. The present first mode provides a method for manufacturing reconstituted plant material comprising the following steps:
extracting a fibrous plant material with a solvent to provide an extract and a fibrous portion;
separating the extract from the fibrous portion;
concentrating the extract to provide a concentrated extract;
forming a base sheet from the fibrous portion;
combining a flavouring contained within a humectant with the concentrated extract;
applying the concentrated extract to the base sheet.

Figure 1 illustrates the preferred embodiment of the first mode, wherein the fibrous plant material is tobacco. With reference to Figure 1 this embodiment is described in detail below.

Any suitable plant material that requires reconstituting as a paper, wherein the loss of volatile compounds during processing (such as alkaloid derivatives or flavour compounds) is detrimental to the final product, may be used in the present first mode.

Preferably, the plant material is tobacco. Any type of tobacco may be used in the present first mode. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

Any suitable parts of the tobacco plant may be used. This includes leaves, stems, roots, bark, seeds and flowers. In particular, tobacco waste is used, tobacco waste is typically in the form of tobacco leaf, stem, powder or dust.

Initially, the tobacco material is added to an aqueous solvent to extract the water soluble tobacco components **1.** Prior to this the tobacco material may be optionally grinded, threshed or cut.

Typically, the amount of aqueous solvent to tobacco material is within the range 75 - 99 wt % of the total weight of the tobacco-aqueous solvent mixture.

The tobacco-aqueous solvent mixture is mechanically beaten during extraction **1** in order to create a pulp.

After extraction **1,** the fibrous portion and aqueous extract are separated, step **2.** This may be achieved by centrifugation, filtration, mechanical pressing or any other methods known in the paper-making industry.

The separated fibrous portion **6** is further refined to form a refined pulp suitable for entry into the paper-making machine **7.** Typical pulp refiners include disk refiners and conical refiners. The refined pulp is then properly slurried and treated to remove contaminants and entrained air.

The refined pulp slurry is applied to a paper-making machine. The paper-making machine comprises a forming section **8,** drainage section, pressing section **9** and a drying section **11.** In the forming section **8** the slurry of refined pulp **7** is applied to a wire sheet, also known as a "forming fabric", to form a web. The wire permits draining of excess water from the web by gravity to form a sheet. Alternatively, suction equipment may also be used permit drainage. Excess water is further removed by means of a press **9.** The press is a typically a pair of squeeze or wringer rolls designed to remove water mechanically and smooth and compress the sheet formed. Drying **11** of the sheet is typically facilitated by drum dryers.

The aqueous extract **3** is concentrated **4.** This is achieved by any conventional means, for example by applying thermal energy and/or evaporation under vacuum.

A flavouring contained within a humectant is combined with the concentrated aqueous extract in step **5.**

The concentrated aqueous extract **5** is preferably applied to the sheet after the sheet has proceeded through the pressing section, and before the sheet has proceeded through the drying section of the paper-making machine, step **10.** The concentrated aqueous extract **5** may be applied to the sheet using spray coating, wetted rollers or any other method suitable in the art. Following this the sheet is dried **11.**

The present first mode also provides a reconstituted tobacco product for a HNB device produced by a process according to the method of the first mode comprising:
a humectant;
a flavouring;
wherein the humectant content is 1 to 50 % by weight of the reconstituted tobacco product,
and wherein the flavouring content is 2 to 25 % by weight of the reconstituted tobacco product.

The reconstituted tobacco product of the present first mode may be used as a tobacco rod, suitable to be ensleeved by a wrapper such as a cigarette paper **12.** The reconstituted tobacco product may be shredded to form a tobacco rod. Alternatively, the reconstituted tobacco product may be wrapped or folded to form a tobacco rod **12.**

The reconstituted tobacco product of the present first mode may also be used as a consumable, or part of a consumable, for an HNB device. It is a reconstituted tobacco product for use in an HNB device.

### Second Mode of Invention: Addition of Nicotine

The present invention provides a method for manufacturing reconstituted plant material comprising the following steps:
extracting a fibrous plant material with a solvent to provide an extract and a fibrous portion;
separating the extract from the fibrous portion;
concentrating the extract to provide a concentrated extract;
forming a base sheet from the fibrous portion;
combining nicotine contained within a humectant with the concentrated extract;
applying the concentrated extract to the base sheet.

Figure 2 illustrates the preferred embodiment of the second mode of invention, wherein the fibrous plant material is tobacco. With reference to Figure 2 this embodiment is described in detail below.

Any suitable plant material that requires reconstituting as a paper, wherein the loss of volatile compounds during processing (such as alkaloid derivatives or flavour compounds) is detrimental to the final product, may be used in the present invention.

Preferably, the plant material is tobacco. Any type of tobacco may be used in the present invention. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

Any suitable parts of the tobacco plant may be used. This includes leaves, stems, roots, bark, seeds and flowers. In particular, tobacco waste is used, tobacco waste is typically in the form of tobacco leaf, stem, powder or dust.

Initially, the tobacco material is added to an aqueous solvent to extract the water soluble tobacco components 1'. Prior to this the tobacco material may be optionally grinded, threshed or cut.

Typically, the amount of aqueous solvent to tobacco material is within the range 75 - 99 wt % of the total weight of the tobacco-aqueous solvent mixture.

The tobacco-aqueous solvent mixture is mechanically beaten during extraction **1'** in order to create a pulp.

After extraction **1',** the fibrous portion and aqueous extract are separated, step **2'.** This may be achieved by centrifugation, filtration, mechanical pressing or any other methods known in the paper-making industry.

The separated fibrous portion **6'** is further refined to form a refined pulp suitable for entry into the paper-making machine **7'.** Typical pulp refiners include disk refiners and conical refiners. The refined pulp is then properly slurried and treated to remove contaminants and entrained air.

The refined pulp slurry is applied to a paper-making machine **8'.** The paper-making machine comprises a forming section **8',** drainage section, pressing section **9'** and a drying section **11'.** In the forming section **8'** the slurry of refined pulp **7'** is applied to a wire sheet, also known as a "forming fabric", to form a web. The wire permits draining of excess water from the web by gravity to form a sheet. Alternatively, suction equipment may also be used permit drainage. Excess water is further removed by means of a press **9'.** The press is a typically a pair of squeeze or wringer rolls designed to remove water mechanically and smooth and compress the sheet formed. Drying **11'** of the sheet is typically facilitated by drum dryers.

The aqueous extract **3'** is concentrated **4'.** This is achieved by any conventional means, for example by applying thermal energy and/or evaporation under vacuum.

Nicotine contained within a humectant is combined with the concentrated aqueous extract in step **5'.**

The concentrated aqueous extract **5'** is preferably applied to the sheet after the sheet has proceeded through the pressing section, and before the sheet has proceeded through the drying section of the paper-making machine, step **10'.** The concentrated aqueous extract **5'** may be applied to the sheet using spray coating, wetted rollers or any other method suitable in the art. Following this the sheet is dried **11'.**

The present invention also provides a reconstituted tobacco product for a HNB device produced by a process according to method of invention comprising:
a humectant;
nicotine;
wherein the humectant content is 1 to 50 % by weight of the reconstituted tobacco product,
and wherein the nicotine content is 2 to 25 % by weight of the reconstituted tobacco product.

The reconstituted tobacco product of the present invention may be used as a tobacco rod, suitable to be ensleeved by a wrapper such as a cigarette paper **12'.** The reconstituted tobacco product may be shredded to form a tobacco rod. Alternatively, the reconstituted tobacco product may be wrapped or folded to form a tobacco rod **12'.**

The reconstituted tobacco product of the present invention may also be used as a consumable, or part of a consumable, for an HNB device. It is a reconstituted tobacco product for use in an HnB device.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A method for manufacturing a reconstituted plant material comprising the following steps:
extracting a fibrous plant material with a solvent to provide an extract and a fibrous portion;
separating the extract from the fibrous portion;
concentrating the extract to provide a concentrated extract;
forming a base sheet from the fibrous portion;
providing a pre-mixture by combining nicotine with a humectant;
combining the pre-mixture with the concentrated extract to provide a modified extract;
applying the modified extract to the base sheet.

2. The method according to claim 1, wherein the solvent is an aqueous solvent.

3. The method according to any preceding claim, wherein the fibrous plant material is tobacco.

4. The method according to claim 3, wherein the tobacco is in the form of tobacco leaf, tobacco stem, tobacco powder, and/or tobacco dust.

5. The method according to any one of claims 1 to 4, wherein the humectant is a polyol.

6. The method according to claim 5, wherein the polyol is propylene glycol or vegetable glycerine or a combination thereof.

7. The method according to any one of claims 1 to 6, wherein the nicotine content of the pre-mixture is within the range 1 to 95 wt % based on the total weight of nicotine and humectant, and optionally within the range 1 to 50 wt %, and optionally within the range 1 to 25 wt %, and optionally within the range 1 to 15 wt %.

8. A reconstituted tobacco product for an HNB device produced by a process according to at least one of the preceding claims comprising:
a humectant;
and nicotine;
wherein the humectant content is 1 to 50 % by weight of the reconstituted tobacco product,
and wherein the nicotine content is 2 to 25 % by weight of the reconstituted tobacco product.

9. The reconstituted tobacco product according to claim 8, wherein the humectant is a polyol.

10. The reconstituted tobacco product according to claim 9, wherein the polyol is propylene glycol or vegetable glycerine or combinations thereof.

11. The reconstituted tobacco product according to any one of claims 8 to 10, wherein the nicotine content is 5 to 25 wt % of the reconstituted tobacco product, and optionally 10 to 25 wt %, and optionally 15 to 25 wt %.

12. The reconstituted tobacco product according to any one of claims 8 to 11, wherein the humectant content is 1 to 40 wt % of the reconstituted tobacco product, and optionally 1 to 20 wt %.

13. Use of the reconstituted tobacco product according to any one of claims 8 to 12 in a HNB device.

## Patentansprüche

1. Verfahren zur Herstellung eines rekonstituierten Pflanzenmaterials, umfassend die folgenden Schritte:
Extrahieren eines faserigen Pflanzenmaterials mit einem Lösungsmittel, um einen Extrakt und einen faserigen Anteil bereitzustellen;
Trennen des Extrakts von dem faserigen Anteil;
Konzentrieren des Extrakts, um einen konzentrierten Extrakt bereitzustellen;
Ausbilden einer Trägerfolie aus dem faserigen Anteil;
Bereitstellen einer Vormischung durch Kombinieren von Nikotin mit einem Feuchthaltemittel;
Kombinieren der Vormischung mit dem konzentrierten Extrakt, um einen modifizierten Extrakt bereitzustellen;
Auftragen des modifizierten Extrakts auf die Trägerfolie.

2. Verfahren nach Anspruch 1, wobei das Lösungsmittel ein wässriges Lösungsmittel ist.

3. Verfahren nach einem der vorhergehenden Ansprüche, wobei das faserige Pflanzenmaterial Tabak ist.

4. Verfahren nach Anspruch 3, wobei der Tabak in Form von Tabakblättern, Tabakstängeln, Tabakpulver und/oder Tabakstaub ist.

5. Verfahren nach einem der Ansprüche 1 bis 4, wobei das Feuchthaltemittel ein Polyol ist.

6. Verfahren nach Anspruch 5, wobei das Polyol Propylenglykol oder pflanzliches Glycerin oder eine Kombination davon ist.

7. Verfahren nach einem der Ansprüche 1 bis 6, wobei der Nikotingehalt der Vormischung innerhalb des Bereichs von 1 bis 95 Gew.-%, bezogen auf das Gesamtgewicht von Nikotin und Feuchthaltemittel, und optional innerhalb des Bereichs von 1 bis 50 Gew.-% und optional innerhalb des Bereichs von 1 bis 25 Gew.-% und optional innerhalb des Bereichs von 1 bis 15 Gew.-% ist.

8. Rekonstituiertes Tabakerzeugnis für eine HNB-Vorrichtung, hergestellt nach einem Verfahren gemäß mindestens einem der vorhergehenden Ansprüche, Folgendes umfassend:
ein Feuchthaltemittel;
und Nikotin;
wobei der Feuchthaltemittelgehalt 1 bis 50 Gew.-% des rekonstituierten Tabakerzeugnisses ist,
und wobei der Nikotingehalt 2 bis 25 Gew.-% des rekonstituierten Tabakerzeugnisses ist.

9. Rekonstituiertes Tabakerzeugnis nach Anspruch 8, wobei das Feuchthaltemittel ein Polyol ist.

10. Rekonstituiertes Tabakerzeugnis nach Anspruch 9, wobei das Polyol Propylenglykol oder pflanzliches Glycerin oder Kombinationen davon ist.

11. Rekonstituiertes Tabakerzeugnis nach einem der Ansprüche 8 bis 10, wobei der Nikotingehalt 5 bis 25 Gew.-% des rekonstituierten Tabakerzeugnisses und optional 10 bis 25 Gew.-% und optional 15 bis 25 Gew.-% ist.

12. Rekonstituiertes Tabakerzeugnis nach einem der Ansprüche 8 bis 11, wobei der Feuchthaltemittelgehalt 1 bis 40 Gew.-% des rekonstituierten Tabakerzeugnisses und optional 1 bis 20 Gew.-% ist.

13. Verwendung des rekonstituierten Tabakerzeugnisses nach einem der Ansprüche 8 bis 12 in einer HNB-Vorrichtung.

## Revendications

1. Procédé de fabrication de matériau végétal reconstitué comprenant les étapes suivantes :
l'extraction d'un matériau végétal fibreux avec un solvant pour obtenir un extrait et une portion fibreuse ;
la séparation de l'extrait de la portion fibreuse ;
la concentration de l'extrait pour obtenir un extrait concentré ;
la formation d'une feuille de base à partir de la portion fibreuse ;
l'obtention d'un prémélange en combinant de la nicotine avec un humectant ;
la combinaison du prémélange avec l'extrait concentré pour obtenir un extrait modifié ;
l'application de l'extrait modifié à la feuille de base.

2. Procédé selon la revendication 1, dans lequel le solvant est un solvant aqueux.

3. Procédé selon une quelconque revendication précédente, dans lequel le matériau végétal fibreux est du tabac.

4. Procédé selon la revendication 3, dans lequel le tabac se présente sous forme de feuille de tabac, de tige de tabac, de poudre de tabac et/ou de poussière de tabac.

5. Procédé selon l'une quelconque des revendications 1 à 4, dans lequel l'humectant est un polyol.

6. Procédé selon la revendication 5, dans lequel le polyol est du propylène glycol ou de la glycérine végétale ou une combinaison de ceux-ci.

7. Procédé selon l'une quelconque des revendications 1 à 6, dans lequel la teneur en nicotine du prémélange est comprise entre 1 et 95 % en poids par rapport au poids total de nicotine et d'humectant, et éventuellement entre 1 et 50 % en poids, et éventuellement entre 1 et 25 % en poids, et éventuellement entre 1 et 15 % en poids.

8. Produit de tabac reconstitué pour un dispositif HNB, obtenu par un procédé selon au moins une des revendications précédentes, comprenant :
un humectant ;
et de la nicotine ;
dans lequel la teneur en humectant est de 1 à 50 % en poids du produit de tabac reconstitué,
et dans lequel la teneur en nicotine est de 2 à 25 % en poids du produit de tabac reconstitué.

9. Produit de tabac reconstitué selon la revendication 8, dans lequel l'humectant est un polyol.

10. Produit de tabac reconstitué selon la revendication 9, dans lequel le polyol est du propylène glycol ou de la glycérine végétale ou des combinaisons de ceux-ci.

11. Produit de tabac reconstitué selon l'une quelconque des revendications 8 à 10, dans lequel la teneur en nicotine est de 5 à 25 % en poids du produit de tabac reconstitué, et éventuellement de 10 à 25 % en poids, et éventuellement de 15 à 25 % en poids.

12. Produit de tabac reconstitué selon l'une quelconque des revendications 8 à 11, dans lequel la teneur en humectant est de 1 à 40 % en poids du produit de tabac reconstitué, et éventuellement de 1 à 20 % en poids.

13. Utilisation du produit de tabac reconstitué selon l'une quelconque des revendications 8 à 12 dans un dispositif HNB.
